# EUROPEAN PATENT SPECIFICATION

(11) **EP 1 536 233 B1**
(45) Date of publication and mention of the grant of the patent: **13.10.2010**
(21) Application number: 03784516.1
(22) Date of filing: 04.08.2003
(51) Int. Cl.: G01N 33/52, G01N 33/68, G01N 33/84

(54) **TEST PIECE FOR PROTEIN ASSAY**
TESTSTÜCK FUR PROTEINASSAY
ELEMENT DE TEST POUR ANALYSE DE PROTEINE

(30) Priority: 09.08.2002 JP 2002233467
(43) Date of publication of application: 01.06.2005
(60) Divisional application: 08020298.9
(73) Proprietor: ARKRAY, Inc., Kyoto-shi, Kyoto 601-8045 (JP)
(72) Inventor: KOSAKA, Hideko, Kyoto-shi, Kyoto 601-8045 (JP)
(74) Representative: Golding, Louise Ann
(86) International application number: PCT/JP2003/009888
(87) International publication number: WO 2004/015423

(56) References cited:
- EP-A- 0 361 244
- WO-A-03/001213
- WO-A-03/075008
- JP-A- 11 508 277
- JP-A- 61 164 158
- JP-A- 62 024 150
- JP-A- 62 024 150
- JP-A- 2002 156 377
- JP-A- 2002 156 377
- TSENG S C G ET AL: "Interaction between rose bengal and different protein components" 1995, CORNEA 1995 UNITED STATES, VOL. 14, NR. 4, PAGE(S) 427-435 , XP002392174 ISSN: 0277-3740 * abstract *

## Description

### TECHNICAL FIELD

This invention relates to a test piece for assaying urine albumin.

### BACKGROUND ART

Assaying the protein in a biological sample is important in pathological diagnosis. For instance, the amount of serum albumin decreases in the case of diminished liver function, while the amount of protein in urine increases in the case of nephritis, nephrotic syndrome, lithiasis, tumors, and other kidney and urinary tract disorders, disorders of the circulatory system and disorders of central nervous system. Therefore, assaying albumin or other proteins can be an important clue in the diagnosis of these disorders.

A simple assay method featuring the use of a protein error indicator is known in the field of protein assay. With this assay method, tetrabromophenol blue (TBPB) is used, for instance, as the protein error indicator. As one example, urine test paper made with TBPB is widely used for primary screening purposes. TBPB changes from yellow to blue through the dissociation of phenolic hydroxyl groups at a pH of about 3 when a protein is present, and therefore can be used to detect protein.

However, test paper made using TBPB as the indicator has inadequate sensitivity with respect to the low protein concentrations of 10 to 20 mg/dL required for clinical use, and is therefore sometimes incapable of detecting protein accurately. For example, in a visual evaluation conducted by comparison with a color chart, the color is very similar between negative protein and trace protein, making it difficult to tell the two apart and hampering accurate evaluation. Meanwhile, when a urine test paper assay apparatus is used, the low sensitivity of TBPB often results in erroneous evaluation.

EP0361244 discloses a test device for detection of albumin in urine samples. The test device comprises a combination of two different indicator dyes which are necessary for performing a sensitive assay. The document discloses Rose Bengal as one possible indicator dye, as well as further ingredients like citrate buffer with a pH of 2-3, but does not disclose the use of a single indicator dye or sensitizers.

JP 2002 156377 relates to erythrosine B for the detection of Bence Jones protein.

JP 62 024150 discloses eosin Y for detecting serum albumin.

The paper by Tseng et al. in Cornea (1995) Vol. 14, p427-435 discloses Rose Bengal as a dye for detecting the protective function of the preoccular mucous tear and does not discuss the use of buffers and sensitizers.

Consequently, there has been a need for a technique that would allow low concentrations of urine albumin to be quantified at higher sensitivity.

### DISCLOSURE OF THE INTENTION

As a result of screening indicators for assaying low concentrations of urine albumin at high sensitivity, the inventors arrived at the present invention upon discovering that a specific halogenated xanthene-based dye is favorable as the targeted indicator.

Specifically, the present invention provides a test piece for assaying urine albumin, containing a halogenated xanthene-based dye, a buffer, and a sensitizer, wherein the halogenated xanthene-based dye is selected from the group of compounds expressed by the following Chemical Formulas (1)-1 and (1)-2, 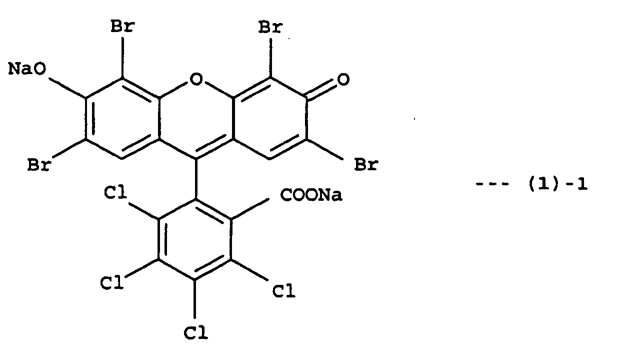 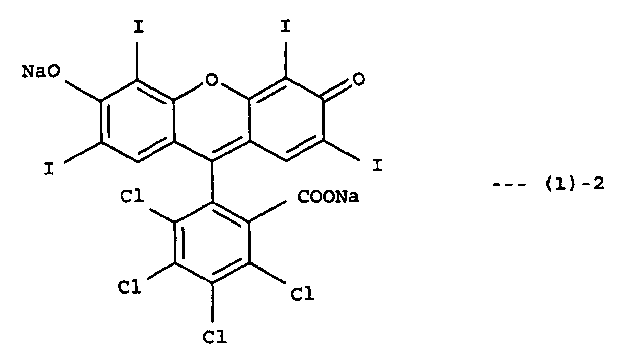 wherein the buffer is selected from the group of glycine buffer, citrate buffer, succinate buffer, malate buffer, and tartrate buffer, and wherein the sensitizer is selected from the group of polyethylene glycol, polypropylene glycol, polycarbonate, and polyvinyl ether; and wherein the pH is set by the buffer at or below the pKa of the dye.

These halogenated xanthene-based dyes are from colorless to light orange in color when no protein is present at a pH at or below the pKa of said dye, but are from red to purple in color when a protein is present. Accordingly, since the original coloring varies from colorless to a pale color, a change in color is easier to detect than when the color changes from yellow to blue as with TBPB. Therefore, if one of the above-mentioned halogenated xanthene-based dyes is used, low-concentration proteins can be detected properly regardless of whether the evaluation is visual or a measurement apparatus is used.

The test piece for assaying urine albumin of the present invention can be manufactured by impregnating an absorbent carrier with an impregnant which contains the abovementioned halogenated xanthene-based dye, a buffer, and a sensitizer, and then drying this product. This test piece can be used directly as it is, or after first being bonded to a non-absorbent material.

There are no particular restrictions on the concentration of the halogenated xanthene-based dye in the impregnant, but it is typically 0.1 to 10 mM, and preferably 0.5 to 2 mM.

The pH of the impregnant is preferably set between 1.5 and 4.5, which is somewhat lower than the pKa of the halogenated xanthene-based dye of the present invention, and is more preferably from 2.0 to 3.5.

Any buffer can be used as long as it has a good buffering action within a pH range of 1.5 to 4.5 and does not impede reaction between the protein and the halogenated xanthene-based dye. Examples of buffers that can be used include glycine buffer, citrate buffer, succinate buffer, malate buffer, and tartrate buffer. There are no particular restrictions on the concentration of the buffer in the impregnant, but it is typically from 0.1 to 1.5 M, and preferably 0.3 to 1 M.

The sensitizer that is used is selected from the group of polyethylene glycol, polypropylene glycol, polycarbonate, and polyvinyl ether. The use of polyethylene glycol or polypropylene glycol is preferred. There are no particular restrictions on the concentration of the sensitizer in the impregnant, but it is typically from 0.05 to 5 wt%, and preferably 0.1 to 1 wt%.

A porous substance containing no protein component can be used as the absorbent carrier, and can be used in the form of a sheet or film, for example. Examples of porous substances include paper-like materials, foams, woven materials, nonwoven materials, and knits. Examples of the material used to form the absorbent carrier include cotton, linen, cellulose, nitrocellulose, cellulose acetate, rock wool, glass fiber, silica fiber, carbon fiber, boron fiber, polyamide, aramid, polyvinyl alcohol, polyvinyl acetate, rayon, polyester, nylon, polyacrylic acid, polyacrylic ester, and polyolefin. There are no particular restrictions on the shape of the absorbent carrier, but it is generally rectangular (either short and wide or long and narrow), circular, or oval.

The non-absorbent material is used in the form of a sheet or film, for example. Examples of the material used to form this non-absorbent material include polyethylene terephthalate, polyester, polypropylene, polyethylene, polyvinyl chloride, polyvinylidene chloride, and polystyrene.

### EXAMPLES

### [Example 1]

In this example, indicators were screened for their ability to detect low concentrations of protein. This screening was performed by adding the test compound such that its concentration in the screening solution would be 0.5 mM, and visually observing the resulting coloration.

The screening solution was prepared by dissolving 15 mg/dL albumin and 0.5 wt% polyethylene glycol in a 0.7 M malate buffer (pH 2.2). The test compounds were various commercially available dyes. As a result, good coloration was seen with the five compounds expressed by the following Chemical Formulas (2)-1 to (2)-5 (compounds (2)-3 to (2)-5, comparative examples). Table 1 shows where these compounds were obtained.

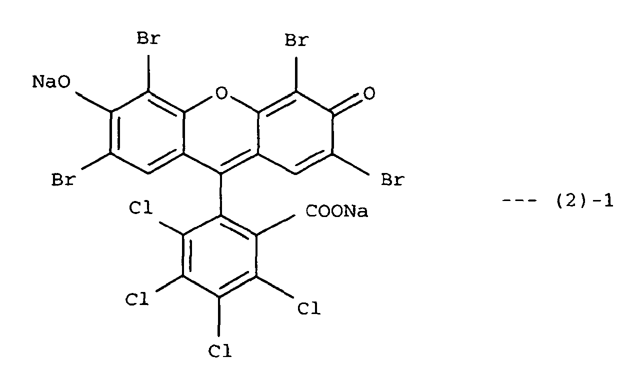

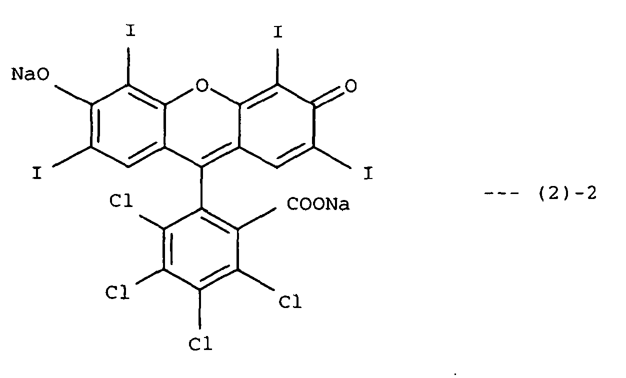

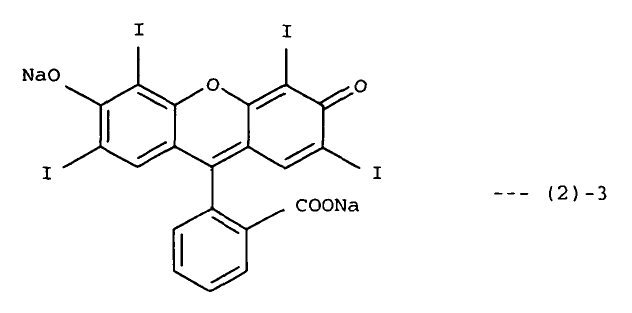

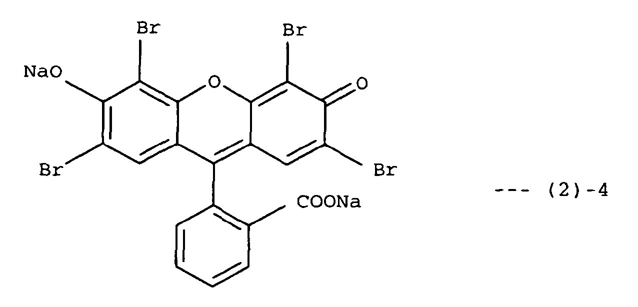

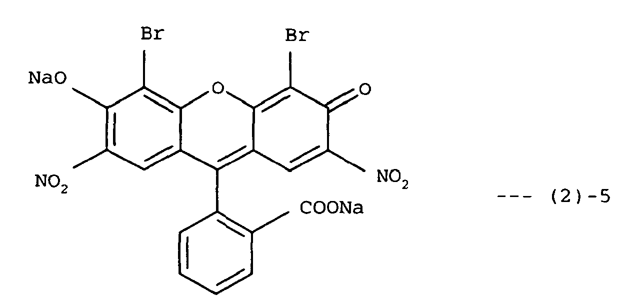

**Table 1**

| Chemical Formula No. | Product name | Manufacturer |
|---|---|---|
| (2)-1 | phloxine B | Tokyo Chemical Industries |
| (2)-2 | rose bengal | Tokyo Chemical Industries |
| (2)-3 | erythrosine B | Tokyo Chemical Industries |
| (2)-4 | eosin Y | Wako Pure Chemical Industries |
| (2)-5 | eosin B | Tokyo Chemical Industries |

### [Example 2]

In this example, the sensitivity of the screened indicators was evaluated. This was accomplished by impregnating each test piece with urine whose albumin concentration was either 0.3 mg/dL (negative) or 15 mg/dL (positive), and measuring the reflectance of each. The test pieces were formed by impregnating filter paper (3MMChr made by Whatman) with an impregnant having the composition given in Table 2. Reflectance was measured with a colorimeter. Table 3 shows the measurement results for the various samples. Table 3 also shows the measurement wavelength for each sample.

**Table 2**

| | Indicator | Buffer | Sensitizer | Solvent |
|---|---|---|---|---|
| Sample 1 | phloxine B (0.5 mM) | malate buffer 0.7 M (pH 2.2) | polyethylene glycol 0.5 wt% | ethanol (40 wt%) |
| Sample 2 | phloxine B (0.5 mM) | malate buffer 0.7 M (pH 2.2) | none | ethanol (40 wt%) |
| Sample 3 | rose bengal (0.5 mM) | malate buffer 0.7 M (pH 2.6) | polyethylene glycol 0.5 wt% | ethanol (40 wt%) |
| Sample 4 | rose bengal (0.5 mM) | malate buffer 0.7 M (pH 2.6) | none | ethanol (40 wt%) |
| Sample 5 | TBPB (0.5 mM) | malate buffer 0.7 M (pH 3.4) | polyethylene glycol 0.5 wt% | ethanol (30 wt%) |
| Sample 6 | TBPB (0.5 mM) | malate buffer 0.7 M (pH 3.4) | none | ethanol (30 wt%) |

| | | | | |
|---|---|---|---|---|
| Note: TBPB = tetrabromophenol blue | | | | |

**Table 3**

| | Measurement wavelength | Reflectance (%) | | Differential Δ |
|---|---|---|---|---|
| | | 0.3 mg/dL (negative) | 15 mg/dL (positive) | |
| Sample 1 | 560 nm | 65.8 | 38.2 | 27.7 |
| Sample 2 | 560 nm | 61.5 | 38.3 | 23.2 |
| Sample 3 | 560 nm | 65.7 | 40.7 | 25.0 |
| Sample 4 | 560 nm | 61.6 | 41.6 | 20.0 |
| Sample 5 | 630 nm | 57.0 | 40.4 | 16.6 |
| Sample 6 | 630 nm | 60.7 | 48.9 | 11.8 |

As is clear from Table 3, with samples 1 and 2 in which the phloxine B expressed by Chemical Formula (2)-1 was used as the indicator, and samples 3 and 4 in which the rose bengal expressed by Chemical Formula (2)-2 was used, the reflectance was higher when the albumin concentration was 0.3 mg/dL (negative) and lower when the albumin concentration was 15 mg/dL (positive) than with samples 5 and 6, in which TBPB was used as the indicator. In other words, phloxine B and rose bengal absorbed less light during non-coloration (negative), and conversely absorbed more light during coloration (positive), than TBPB because they were colorless under these pH conditions. Accordingly, phloxine B and rose bengal have a large reflectance differential Δ between negative urine and positive urine, with this differential being about twice that with TBPB. Therefore, phloxine B and rose bengal can be said to have higher sensitivity with respect to albumin, and allow albumin to be properly detected even when the albumin concentration is low (about 10 to 20 mg/dL).

The inventors also checked whether the samples could be evaluated visually. As a result, samples 1 to 4, in which phloxine B and rose bengal were used, changed from colorless to red when the albumin concentration was 15 mg/dL, and this coloration (positive) could be easily confirmed. In contrast, with samples 5 and 6, in which TBPB was used, there was almost no difference from the negative yellow color when the albumin concentration was 15 mg/dL, making it very difficult to visually discern any coloration. Thus, when phloxine B and rose bengal were used as the indicator, albumin could be easily detected visually, even at a low albumin concentration.

## Claims

1. A test piece for assaying urine albumin, containing a halogenated xanthene-based dye, a buffer, and a sensitizer,
wherein the halogenated xanthene-based dye is selected from the group of compounds expressed by the following Chemical Formulas (1)-1 and (1)-2, 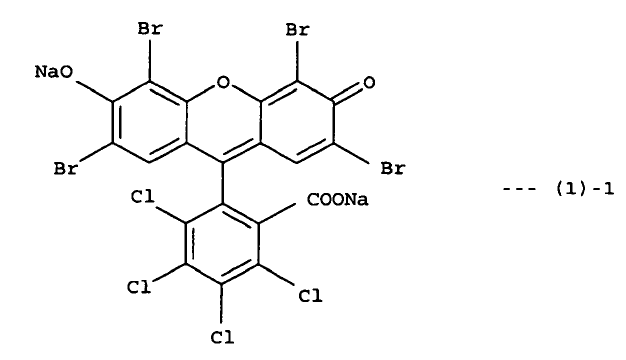 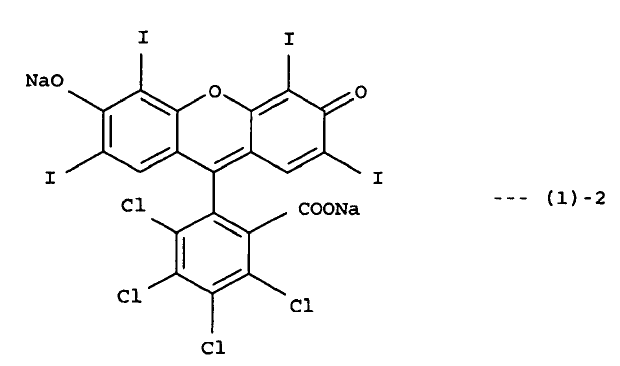 wherein the buffer is selected from the group of glycine buffer, citrate buffer, succinate buffer, malate buffer, and tartrate buffer, and
wherein the sensitizer is selected from the group of polyethylene glycol, polypropylene glycol, polycarbonate, and polyvinyl ether; and
wherein the pH is set by the buffer at or below the pKa of the dye.

## Patentansprüche

1. Teststück zum Untersuchen von Urinalbumin, das einen halogenierten Farbstoff auf Xanthenbasis, einen Puffer und einen Sensibilisator enthält,
wobei der halogenierte Farbstoff auf Xanthenbasis aus der Gruppe von Verbindungen ausgewählt ist, die durch die folgenden chemischen Formeln (1)-1 und (1)-2 ausgedrückt werden 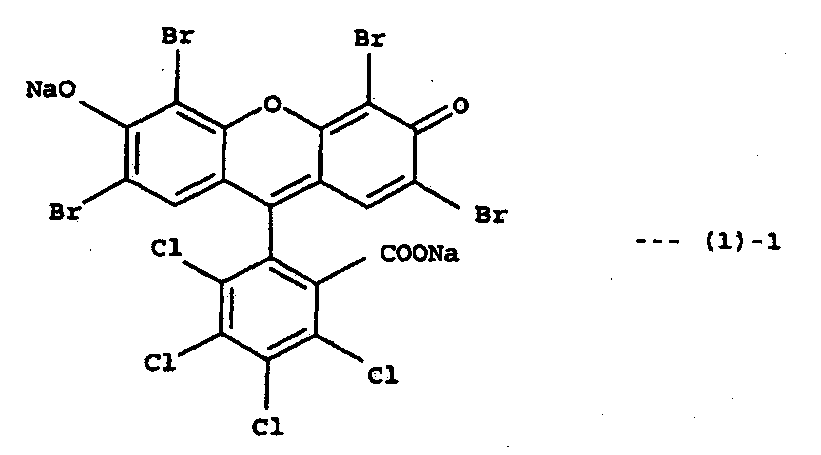 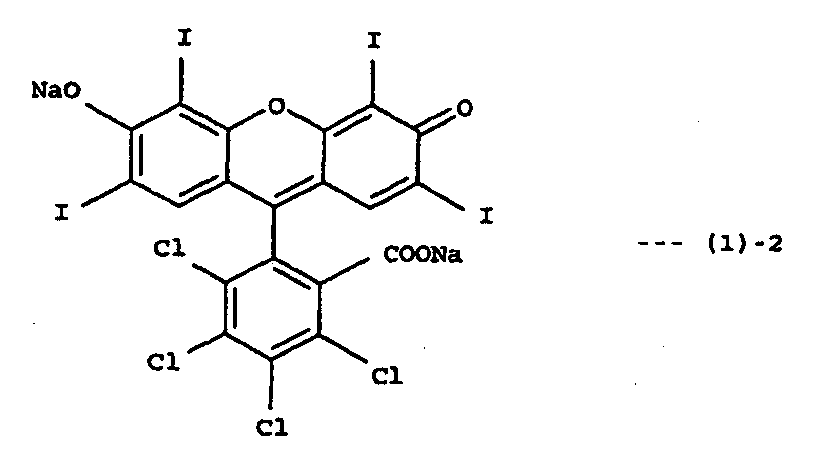 wobei der Puffer aus der Gruppe ausgewählt ist, die Glycin-Puffer, Citrat-Puffer, Succinat-Puffer, Malat-Puffer und Tartrat-Puffer enthält, und
wobei der Sensibilisator aus der Gruppe ausgewählt ist, die Polyethylenglycol, Polypropylenglycol, Polycarbonat und Polyvinylether enthält; und
wobei der pH-Wert durch den Puffer auf oder unter den pKa-Wert des Farbstoffs eingestellt wird.

## Revendications

1. Eprouvette d'essai pour analyser de l'albumine urinaire, contenant un colorant à base de xanthène halogéné, un tampon et un sensibilisateur,
dans laquelle le colorant à base de xanthène halogéné est choisi parmi le groupe constitué des composés exprimés par les formules chimiques (1)-1 et (1)-2 suivantes, 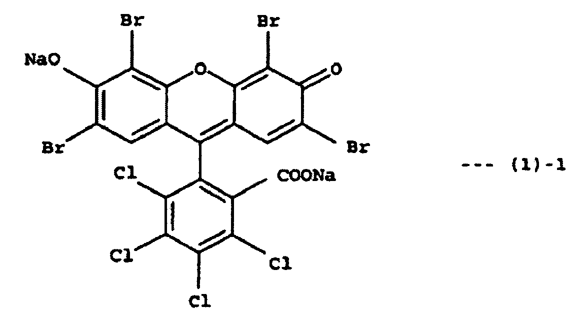 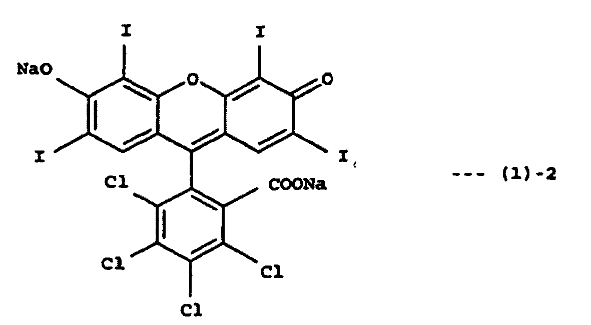 dans laquelle le tampon est choisi parmi le groupe constitué d'un tampon glycine, d'un tampon citrate, d'un tampon succinate, d'un tampon malate et d'un tampon tartrate, et dans laquelle le sensibilisateur est choisi parmi le groupe constitué de polyéthylène glycol, de polypropylène glycol, de polycarbonate et de polyvinyl éther ; et
dans lesquelles le pH est fixé par le tampon au pKa du colorant, ou en dessous.
